Europäisches Patentamt

European Patent Office

Office européen des brevets

(11) Veröffentlichungsnummer : **0 069 259**
**B1**

# (12) EUROPÄISCHE PATENTSCHRIFT

(45) Veröffentlichungstag der Patentschrift :
09.01.85

(21) Anmeldenummer : 82105329.5

(22) Anmeldetag : 18.06.82

(51) Int. Cl.⁴ : **A 61 K  9/22**, A 61 K  9/52,
A 61 K 31/135

(54) **Bromhexin-Retardform und Verfahren zu ihrer Herstellung.**

(30) Priorität : 07.07.81 DE 3126703

(43) Veröffentlichungstag der Anmeldung :
12.01.83 Patentblatt 83/02

(45) Bekanntmachung des Hinweises auf die Patenterteilung : 09.01.85 Patentblatt 85/02

(84) Benannte Vertragsstaaten :
AT BE CH DE FR IT LI LU NL SE

(56) Entgegenhaltungen :
EP-A- 0 032 562
DE-A- 2 148 391
DE-A- 2 336 218
DE-A- 2 415 490
GB-A-  968 254
GB-A- 1 464 082
CHEMICAL ABSTRACTS, Band 89, Nr. 2, 10. Juli 1978,
Seite 366, Nr. 12078g, Columbus, Ohio, USA M.
DITTGEN et al.: "Drug release from embedding forms
based on polyacrylate"

(73) Patentinhaber : **Dr. Karl Thomae GmbH**
**Postfach 1755**
**D-7950 Biberach (Riss) (DE)**

(72) Erfinder : **Gruber, Peter, Dr. Dipl.-Chem.**
**Wetterkreuzstrasse 36**
**D-7950 Biberach 1 (DE)**
Erfinder : **Schmid, Jochen, Dr. Dipl.-Chem.**
**Panoramaweg 19**
**D-7951 Warthausen 1 (DE)**
Erfinder : **Lechner, Horst, Dr. Dipl.-Chem.**
**Nickeleshalde 8**
**D-7950 Biberach 1 (DE)**
Erfinder : **Bauer, Eckhart, Dr. Dipl.-Biochem.**
**Nickeleshalde 11**
**D-7950 Biberach 1 (DE)**

**Beschreibung**

Die Erfindung betrifft mit einem Überzug versehene, Bromhexin und Salze des Bromhexins enthaltende Arzneiformen und Verfahren zu ihrer Herstellung. Diese oral zu verabreichenden Formen geben, da der Überzug wie eine Dialysemembran wirkt, den Wirkstoff im Magen-Darm-Trakt in geregelter Weise verzögert ab.

Bei Bromhexin, N-Cyclohexyl-N-methyl-(2-amino-3,5-dibrombenzyl)-amin, und seinem Hydrochlorid handelt es sich um einen seit Jahren bewährten Wirkstoff. Die therapeutische Wirkung von Bromhexin bei Krankheiten des Respirationstraktes, die mit pathologisch veränderter Sekretbildung einhergen, wurde durch Einfach- und Doppelblindprüfung vielfach bestätigt. Neben dieser Wirkung beeinflußt Bromhexin auch den Husten selbst. Die Hustenfrequenz, das Integral der Hustenstöße und die Lautstärke des Hustens werden positiv beeinflußt. Stets empfinden die Patienten eine Erleichterung der Atmung. Deshalb würde eine Retardform mit verlängerter Wirkung gerade in der Nacht für den Patienten große Vorteile bieten, da sie die subtherapeutischen Blutspiegel der unverzögerten Bromhexin-Arzneiformen infolge des großen Einnahmeintervalls über Nacht vermeidet.

Neben den jährlich immer wiederkehrenden akuten Tracheobronchitiden hat sich Bromhexin auch bestens bei chronischen Bronchitiden, Asthmabronchiale und chronischen Sinusitiden bewährt, deren Heilung und Linderung lange Behandlungszeiträume erfordern. Auch hier bringt eine Verringerung der Zahl der Einnahmen pro Tag eine Verbesserung der Compliance der Patienten, was bekanntlich bei einer Langzeitmedikation von besonderer Bedeutung ist. Aufgrund dieser offensichtlichen Vorteile einer Retardform von Bromhexin hat es daher nicht an Versuchen zu ihrer Realisierung gefehlt.

Man mußte jedoch feststellen, daß selbst mikronisiertes Bromhexin hydrochlorid, welches über eine magensaftresistente Form in den Intestinaltrakt eingebracht wurde, zu keiner nennenswerten Resorption führte. In solchen Fällen hat man schon wiederholt Erfolge erzielt, wenn man den Wirkstoff mit einem Emulgator vermischte, der dann im Darmsaft den Wirkstoff in solubilisierter Form freisetzt. Jedoch auch mit dieser Form konnte keine Resorption aus dem Darm realisiert werden.

Im allgemeinen muß ein Wirkstoff für die Entwicklung einer wirksamen Retardform folgende Voraussetzungen erfüllen :

— gute, pH-unabhängige Löslichkeit im gesamten Magen-Darm-Trakt.
— keine Änderung der Resorptionsgeschwindigkeit im resorptionsfähigen Teil des Magen-Darm-Traktes.

Es zeigt sich, daß Bromhexin keine der genannten Voraussetzungen erfüllt :

— Es ist in wäßrigem Milieu nur im Sauren löslich, oberhalb pH 4 ist die Substanz praktisch wasserunlöslich. Dies bedeutet, daß Bromhexin nur im oberen Gastrointestinaltrakt gelöst und damit auch resorbiert werden kann, während es bei den im Darmbereich auftretenden höheren pH-Werten unlöslich bleibt und nicht resorbiert wird.
— Da die Passagezeit durch den Magen und den obersten Darmbereich (mit ausreichend saurem pH) relativ kurz ist (ca. 0,5-1 Stunde), ist es daher schwierig, eine Resorption über mehrere Stunden zu erreichen. Außerdem kann die Verweildauer im Magen und in den verschiedenen Darmabschnitten sehr stark variieren, d. h. inter- und intraindividuelle Blutspiegelschwankungen sind naturgemäß bei einer Substanz, deren Löslichkeit vom pH abhängt, extrem groß, wenn die Substanzzubereitung eine langsame Freigaben, wie sie für Retardformen notwendig ist, aufweist.
— Selbst wenn Bromhexin gelöst in verschiedene Darmabschnitte eingebracht wird, nimmt das Ausmaß der Resorption vom Duodenum zum Colon hin stark ab.

Normalerweise bereitet es keine Schwierigkeiten, bei gut löslichen und weitgehend pH-unabhängig gut löslichen Wirkstoffen Depotpräparate zu realisieren. Retardeigenschaften werden beispielsweise dadurch erzielt, daß

1. der Wirkstoff zusammen mit Hilfsstoffen so formuliert wird, daß er nur sehr langsam freigegeben wird, z. B. durch Einbetten in eine sich sehr langsam auflösende Matrix ;
2. der Wirkstoff zusammen mit Hilfsstoffen zu Tabletten, Pellets usw. geformt wird, die dann mit einem Überzug versehen werden, der eine langsame Freisetzung des Wirkstoffs zur Folge hat.

Es hat sich nun gezeigt, daß sich selbst bei Einsatz von gut löslichen Salzen des Bromhexins keine Retardform realisieren läßt, da durch saure Salze allein die extreme pH-Abhängigkeit der Löslichkeit von Bromhexin nicht überwunden werden kann. Bringt man beispielsweise Bromhexin-hydrochlorid in die Form einer Matrix-Depottablette (Beispiel 11), so kann aus dieser Form Bromhexin nur solange gelöst freigegeben werden, wie sich die Tablette im sauren Milieu des Magens befindet. Gelangt die Matrixtablette in den Dünndarm, so kommen die Wirkstofffreigabe und damit die Resorption praktisch zum Erliegen. Der Darmsaft puffert augenblicklich die sauren Salze zur praktisch unlöslichen Bromhexin-Base um. Die Löslichkeit der Bromhexin-Base in Phosphat-Puffer beträgt

pH 4,5     0,065 %
    5,0     0,015 %
    6,0     0,003 %
    6,5     0,001 %
    7,0     0,0003 %

Verständlicherweise hielt der Fachmann aufgrund dieser extrem niedrigen Löslichkeiten und der genannten in vivo-Vorversuche die Entwicklung einer funktionierenden Depotform für ausgeschlossen, setzt sie doch voraus, daß auch im pH-Bereich des Intestinaltraktes Bromhexin in gelöster, resorptionsfähiger Form über Stunden zur Verfügung stehen müßte.

Es ist nun überraschenderweise gelungen, eine Bromhexin-Retardform zu finden, die den besonderen physikalischen und biochemischen Eigenschaften des Bromhexins voll Rechnung trägt und einen lang anhaltenden Bromhexin-Blutspiegel gewährleistet.

Dies ist dadurch gelungen, daß mehrere Technologien in geeigneter Weise angewendet wurden, deren Kombination zu einem für den Fachmann nicht vorhersehbaren positiven Ergebnis führte.

Folgende Prinzipien wurden dabei angewendet :

1. Die Unlöslichkeit des Bromhexins bei höherem pH im Intestinaltrakt wird durch den Zusatz von sauer wirkenden Stoffen überwunden.

2. Die aus Bromhexin bzw. Bromhexin-Salz und den sauer wirkenden Stoffen gebildeten Arzneiformen werden mit einer Membran umgegeben, die einerseits eine speziell für Bromhexin angepaßte Freigabecharakteristik zeigt, andererseits aber auch die sich normalerweise viel schneller auflösen sauren Stoffe ausreichend lange innerhalb der Arzneiform zurückhält.

Gegenstand der Erfindung sind :

a. aus Bromhexin und/oder dessen Salzen bestehende sphäroide Teilchen, die mit einer Diffusionsmembran überzogen sind und die bevorzugt in Hartgelatinekapseln abgefüllt werden,

b. oder Tabletten, die durch Verpressen solcher, wie unter a) beschriebener Teilchen erhalten wurden, und

c. Tabletten, die aus Bromhexin und/oder dessen Salzen und sauren Substanzen durch Granulierung dieser Gemisches und durch anschließendes Verpressen des Granulats hergestellt und mit einer Diffusionsmembran überzogen sind.

Die Bromhexin-Retardformen (a) und (b) bestehen aus einer Reihe sphäroider Teilchen gleicher oder verschiedener Teilchengröße, die sich ihrerseits aus Bromhexin und/oder aus Salzen des Bromhexins und Säuren, z. B. organischen Genußsäuren, im Verhältnis von mindestens 2 Mol Säure auf 1 Mol Bromhexin-Base bzw. 1 Mol Säure auf 1 Mol Bromhexin-Salz zusammensetzen, wobei der Säureanteil aber auch wesentlich höher sein kann, beispielsweise bis 75 Mol, vorzugsweise jedoch 5-25 Mol, und einer die sphäroiden Teilchen umgebenden Diffusionsmembran, bestehend im wesentlichen aus säureunlöslichen, darmsaftlöslichen Lacken, wobei der Hüllenanteil zweckmäßigerweise 2 bis 30 Gewichtsprozent, vorzugsweise jedoch 3 bis 20 Gewichtsprozent, bezogen auf das Gewicht der sphäroiden Teilchen, beträgt. Das Verhältnis Bromhexin/Säure muß so gewählt werden, daß eine vollständige Freisetzung des Wirkstoffes in gelöster Form unabhängig vom pH-Milieu im Magen-Darmtrakt gewährleistet ist.

Zur Herstellung der Arzneiformen wird beispielsweise Bromhexin bzw. Salze des Bromhexins mit Säuren, z. B. organischen Genußsäuren, wie Zitronensäure oder Weinsäure, in einem der oben angegebenen Verhältnisse gemischt und granuliert. Sphäroide Teilchen können aber auch in Form größerer Kristalle mit sphäroider Gestalt (z. B. bei der Verwendung von kristallisierten Salzen des Bromhexins) in Form ausgerundeter Granulate oder in Form kleiner Perlen, sogenannter Pellets, vorliegen. Die Herstellung solcher Formen erfolgt nach an sich bekannten Verfahren. Bevorzugt sind kleine Perlen, deren Größe zwischen 0,1 und 3 mm Durchmesser, vorzugsweise zwischen 0,8 und 1,5 mm Durchmesser liegt. Als Säuren eignen sich eine Vielzahl von toxikologisch unbezdenklichen Säuren wie z. B. Fumarsäure, Äpfelsäure, Weinsäure, Zitronensäure, Bernsteinsäure, Ascorbinsäure oder Gemische dieser Säuren, aber auch saure Salze wie z. B. Natrium- oder Kaliumhydrogensulfat oder die Mononatrium- bzw. -kaliumsalze der Weinsäure oder Zitronensäure. Es können als Starterkerne auch andere Säuren bzw. sauer reagierende Stoffe verwendet werden wie für die aufzutragende Pulvermischung, d. h. es können auf Starterkerne, die aus den oben genannten organischen Genußsäuren und den sauer reagierenden Stoffen bestehen, jeweils andere Säuren und sauer reagierende Stoffe im Gemisch mit Bromhexin bzw. Bromhexin-Salzen in der oben beschriebenen Weise aufgetragen werden. Außerdem kann die saure Komponente des auf die Kerne aufzutragenden Gemisches aus mehreren der oben genannten Säuren und sauer reagierenden Stoffe bestehen.

Für die Starterkerne sind jene sauren Stoffe besonders geeignet, die annähernd kugelförmige Gestalt aufweisen, z. B. Weinsäure, Zitronensäure, Äpfelsäure, Bernsteinsäure, Ascorbinsäure, Na- oder Kaliumhydrogensulfat und Mono-Natrium oder -Kaliumsalze der mehrbasischen Säuren. Es können aber auch neutrale Verbindungen wie Bernsteinsäureanhydrid verwendet werden, die nach Hydrolyse sauer reagieren.

Durch das Vorliegen sphäroider Teilchen, z. B. ausgerundeter Granulatkörner oder Pellets, wird die Wirkstoffesamtdosis auf viele hundert selbständige, kleine Retardformen aufgeteilt. Damit wird eine statistisch gleichmäßige, weitgehend reproduzierbare Passage der kleinen, mit der Diffusionsmembran umgebenen Retardformen durch den Magen-Darm-Trakt gewährleistet.

Die Teilchen bzw. Pellets werden, wie oben bereits erwähnt, nach an sich bekannter Verfahren erzeugt, beispielsweise mit Hilfe von Dragieranlagen nach dem Merumeriza-Verfahren aus Säure- und Bromhexinpulver unter Zuhilfenahme von Klebstofflösungen. Die Herstellung erfolgt auch beispielsweise unter Benützung eines Pelletiertellers bzw. von Feuchtmischgeräten mit speziellen Rührarmen. Bevorzugt erzeugt man jedoch die Pellets durch Auftragen des Wirkstoffes oder einer Wirkstoffsuspension auf Starterkerne, die aus den bereits beschriebenen geeigneten Säuren bestehen, in Gegenwart eines Haftmittels. Geeignete Haftmittel sind Klebstofflösungen, wie Stärkekleister, Zuckersirup und Lösungen von Gelatine, Guar-Gummi, Celluloseäther (z. B. Methyl-, Äthyl-, Hydroxyäthyl-, Hydroxypropylmethylcellulose) oder Polyvinylpyrrolidon.

Im einzelnen geht man bei einer bevorzugten Verfahrensmethode so vor, daß man ausgerundete Starterkerne von Weinsäure mit einem mittleren Durchmesser von 0,3-1 mm, vorzugsweise jedoch von 0,7-0,9 mm, in einem geeigneten Kessel gleichmäßig mit einer alkoholischen Polyvinylpyrrolidonlösung besprüht und mit Teilen einer Mischung aus 8 Teilen Bromhexin, 1 Teil Weinsäure und 1 Teil Talcum versetzt bis die Kügelchen wieder frei rollen. Nach dem Trocknen wird dieser Vorgang sooft wiederholt, bis die gesamte Mischung aufgetragen ist. Die entstehenden Bromhexin-Pellets haben eine Größe von 0,8-1,0 mm und bestehen vorzugsweise zu mehr als 90 % aus Wirkstoff und Säure. Es ist aber auch möglich, den Wirkstoff in der Klebstofflösung zu lösen bzw. zu suspendieren und diese Lösung oder Suspension gleichmäßig auf die Oberfläche der Starterkerne aufzusprühen.

Zur Herstellung der erfindungsgemäßen Retardtabletten (c) wird im allgemeinen zuerst Bromhexin oder dessen Salze mit den genannten sauren Substanzen in den beschriebenen Molverhältnissen gemischt, feucht oder trocken granuliert, und nach Sieben und gegebenenfalls nach Zusatz weiterer Hilfsstoffe, z. B. Magnesiumstearat, zu Tabletten von beispielsweise 4 bis 13 mm Durchmesser verpreßt. Die so hergestellten Tabletten werden anschließend mit einem Lack überzogen, dessen Zusammensetzung später näher beschrieben wird.

Umfangreiche in-vitro- und in-vivo-Versuche haben gezeigt, daß dem Diffusionslack eine wesentliche Bedeutung zukommt. Der auf den Retardformen aufgesprühte Lack darf sich im resorptionsähigen Teil des Gastrointestinaltraktes nicht auflösen, die Hülle muß solange im Intestinaltrakt erhalten bleiben, bis der gesamte Wirkstoff herausdiffundiert ist. Die Hülle muß die im Kern befindliche Säure solange zurückhalten, bis das darin sich befindende Bromhexin gänzlich aufgelöst ist. Geht die Hülle vorzeitig in Lösung bzw. wird sie undicht, so dringt der im großeb Überschuß vorhandene Darmsaft in das Innere der Bromhexin-Retardformen ein und neutralisiert die dort vorhandene Säure. Damit kann wegen der praktischen Unlöslichkeit des Bromhexins im pH-Bereich des Darmes kein Wirkstoff mehr in Lösung gehen und resorbiert werden. Die im Inneren der Retardformen vorhandene Säure, die sich in der diffundierenden Flüssigkeit auflöst, löst ihrerseits das Bromhexin auf und schleppt dieses durch die Membran. Durch die Steigerung der Durchlässigkeit der Diffusionsmembran im Intestinaltrakt wird in den tieferen Bezirken des Darmtraktes vermehrt saure Wirkstofflösung abgegeben. Damit wird die abnehmende Resorptionsgeschwindigkeit in tieferen Darmabschnitten durch das gesteigerte Angebot an diffundierter Wirkstofflösung ausgeglichen. Dieser Vorgang kann auch noch in anderer Weise elegant realisiert werden :

So besprüht man zunächst den bei der Herstellung des Granulates für die Diffusionstabletten bzw. für die Herstellung der Pellets eingesetzten Säure-Starterkern mit einer Lösung eines retardierenden Lackes (siehe Beispiel 5). Damit wird erreicht, daß sich das Angebot an Säure zur Auflösung des Bromhexins nicht erschöpft bzw. bei Verwendung eines darmsaftlöslichen Lackes allmählich steigert.

Es war überraschend und absolut nicht vorhersehbar, daß die aus der Zubereitung austretende saure Bromhexinlösung über längere Zeit resorbierbar bleibt, obgleich z. B. selbst mikronisiertes Bromhexin und mit Emulgatoren solubilisiertes Bromhexin, die in den Intestinaltrakt eingebracht wurden, praktisch keine Resorption zeigten.

Ursache dieses unerwarteten Effekts könnte entweder eine auch in-vitro zu beobachtende übersättigte Bromhexin-Darmsaftlösung sein oder das Bromhexin fällt nach der Diffusion im Darmsaft annähernd moleculardispers aus und steht in dieser fein verteilten Form weiterhin zur Resorption zur Verfügung.

Grundsätzlich könnten die Dialysemembranen der Bromhexin-Arznneiformen aus den üblichen magen- und darmsaftunlöslichen Retardlacken wie Äthylcellulose, Acetylcellulose oder Lacksubstanzen auf Acrylat- bzw. Methacrylatbasis (bekannt unter den Handelsnamen Eudragit retard S und Eudragit retard L) bestehen. Da gelöstes Bromhexin jedoch unmittelbar nach Verlassen des Magens im obersten Darmabschnitt besonders rasch resorbiert wird, was zu hochschießenden Blutspiegelspitzen führen kann, andererseits in den tieferen Darmabschnitten die Resorptionsgeschwindigkeit deutlich abnimmt, ist eine solche Retardhülle zur Schaffung von Arzneiformen mit langanhaltenden Blutspiegeln bei hoher Bioverfügbarkeit unbrauchbar.

Es wurde nun gefunden, daß man diese Nachteile dadurch vermeidet, daß man die Diffusionsmembran aus überwiegend darmsaftlöslichen und magensaftunlöslichen Lacken aufbaut. Dadurch erreicht man, daß der Wirkstoff zuerst verzögert und in den tieferen Darmabschnitten beschleunigt freigegeben wird.

Unter anderen hat sich eine Zusammensetzung von 50 Gewichtsprozent eines Methacrylsäure-Methacrylsäureester-Mischpolymerisats (Säurezahl 180-200), bekannt unter dem Handelsnamen Eudragit S, und 50 Gewichtsprozent Hydroxypropylmethylcellulosephthalat, bekannt unter dem Handelsnamen HP 55 (beides sogenannte darmsaftlösliche Lacke), als besonders vorteilhaft erwiesen. Man verwendet zum Besprühen der Arzneiformen beispielsweise eine 10 bis 15 %ige Lösung beider Komponenten. Obwohl eine derartige Umhüllung nur aus sogenannten darmsaftlöslichen Komponenten besteht, kommt es im resorptionsfähigen Teil des Intestinaltraktes überraschenderweise nicht zur Auflösung der Hülle. Daß derartige Hüllen überhaupt möglich, sogar in diesem Fall besonders geeignet sind, ist aus zwei Gründen überraschend :

1. In der DB-PS 24 15 490 und der DT-AS 23 36 218 wird ausgeführt, daß zur Erzeugung einer stabilen Hülle ein Anteil von mindestens 30 bzw. 15 % in Magen- und Darmsaft unlöslicher Hüllenbestandteile notwendig sei.

2. Es war nicht vorhersehbar, daß selbst bei 100 % darmsaftlöslichem Lack überhaupt eine Diffusion zustande kommt, da ja ständig Säure im Innern der Arzneiform gelöst wird und durch die Membran diffundiert.

Als darmsaftlösliche Lackkomponenten kommen neben den oben genannten alle sogenannten magensaftresisten Lacke wie z. B. Celluloseacetatphthalat, Äthylcellulosephthalat, Hydroxypropylmethylcellulosesuccinat, Celluloseacetatsuccinat, Hydroxypropylmethylcellulosehexadrophthalat, Celluloseacetathexahydrophthalat, Hydroxypropylmethylcellulosetrimellitat oder Methacrylsäure-Methacrylsäurester-Mischpolymerisat (Säurezahl 300 bis 330, auch Eudragit L genannt) für sich oder in Gemischen miteinander in Frage.

Man kann einen gewissen Teil der darmsaftlöslichen Lacke auch durch sowohl im Magen als auch in Darm unlösliche Lacke ersetzen, ohne daß dadurch die optimal wirkende Retardform in ihrer Wirkung allzu große Einbußen erleidet. Als solche Lacke eignen sich Lacksubstanzen auf Acrylat. Methacrylatbasis, die unter dem Handelsnamen Eudragit retard S und Eudragit retard L bekannt sind. Hierbei können die säure- und darmsaftunlöslichen Lackkomponenten bis zu 50 Gewichtsprozent, vorzugsweise bis zu 30 Gewichtsprozent betragen, im Falle von Äthylcellulose bis zu 14 Gewichtsprozent.

Zusammengefaßt kann festgestellt werden, daß die ungewöhnlichen Eigenschaften von Bromhexin (extrem stake pH-Abhängigkeit der Löslichkeit, nahezu unlöslich ab pH 4,O) einen spezifisch angepaßten Dialysemembrantyp, der eine gewisse pH-abhängige Freigabesteuerung ermöglicht, erfordern. Die Zusammensetzung der Hülle wird so gewählt, daß sie im pH-Bereich bis 4,0 eine Freigabeverzögerung und darüber mit steigendem pH eine sich beschleunigende Wirkstofffreisetzung zeigt. Die Verweilzeit von Arzneimitteln und der pH-Wert im Magen und in den verschiedenen Darmabschnitten sind jedoch von Person zu Person und bei einer Person zu verschiedenen Zeiten stark unterschiedlich.

Eine pH-Abhängigkeit der Freigabe hätte daher auch große Unterschiede im zeitlichen Verlauf der Blutspiegel zur Folge. Teilt man jedoch die Gesamtdosis an Bromhexin auf hunderte von selbstständigen, kleinen Retardformen auf, so wird eine statistisch gleichmäßige, weitgehend reproduzierbare Passage dieser Retardform durch den Magen-Darm-Trakt gewährleistet. Die Auswirkungen der Unterschiede im pH-Gefälle und der Magen-Darmmotilität bei den einzelnen Patienten auf den Bromhexin-Blutspiegelverlauf werden dadurch weitgehend ausgeglichen. Die Realisierung des Prinzips einer gewissen pHabhängigen Freigabesteuerung favorisieren daher bei Bromhexin die Verwendung von sphäroiden Teilchen, wie ausgerundete Granulate oder Pellets vor bikonvexen, mit einer Dialysemembran überzogenen Tabletten.

Die erfindungsgemäß genannten Überzüge bzw. Umhüllungen können die üblichen Hilfsstoffe, wie Weichmacher, Netzmittel und Farbstoffe enthalten. Geeignet sind pharmakologisch unbedenkliche Weichmacher wie z. B. solche aus der Reihe der Phthalsäure-, Phosphorsäure- oder Zitronensäureester und Polyäthylenglykole, vorzugsweise wird Glycerintriacetat und Rizinusöl verwendet.

Das Aufbringen der Dialysemembran auf die Arzneiformen erfolgt nach an sich bekannten Methoden. Dies kann in einem rasch rotierenden Kessel oder im Wirbelschichtverfahren durch Aufsprühen der die Diffusionsmembran bildenden Lacklösung erfolgen.

Der Dosisbereich für den Wirkstoff Bromhexin liegt zwischen 5 und 100 mg, vorzugsweise jedoch zwischen 15 bis 60 mg. Die nach den oben beschriebenen Verfahren vorzugsweise hergestellten sphäroiden Teilchen werden, nachdem sie mit einer Diffusionsmembran versehen sind, z. B. in Hartgelatine-Kapseln abgefüllt. Dabei ist es möglich, Pellets bzw. Teilchen verschiedener Verzögerungsstufen zu mischen und gegebenenfalls auch unverzögerte Wirkstoffteilchen bzw. -pellets als sogenannte Startdosis hinzuzufügen. Die Bromhexin-Retardteilchen können aber auch mit anderen pharmazeutischen Hilfsstoffen gemischt und zu Tabletten verpreßt werden.

Dies ist bei Teilchen bzw. Pellets mit einem Durchmesser unter 1 mm ohne nennenswerte Beschädigung der Diffusionsmembran möglich. Eine solche Tablette zerfällt nach dem Einnehmen in wenigen Sekunden und gibt, wie die Kapseln, die sphäroiden Bromhexin-Retardteilchen frei (Beispiel 10).

Durch die Auffindung der erfindungsgemäßen Bromhexin-Darreichungsformen wurden folgende Probleme überwunden:

a) Es gelang die Schaffung eines Prinzips, durch welches Bromhexin überhaupt löslich und unabhängig in seiner Löslichkeit von den pH-Werten des Gastrointestinaltraktes wird.

b) Es wurde eine Diffusionshülle gewählt, die die Säure vor dem vorzeitigen Abpuffern durch den im riesigen Überschuß vorhandenen Darmsaft über Stunden schützt. Die Hülle sorgt für das vollständige Austreten des gelösten Bromhexins aus den Retardpellets und gleicht die Unterschiede der Bromhexin-Resorptionsgeschwindigkeiten in den einzelnen Abschnitten des Gastrointestinaltraktes durch eine zunächst verzögerte, dann sich beschleunigende Freigabe bei weiterem Vordringen in die tieferen Abschnitte des Intestinaltraktes aus; die gefundene Hülle bewirkt deshalb anstelle einer linearen Freigabe des Wirkstoffen eine pH-abhängige Freigabe. Durch die Verteilung des Wirkstoffes vorzugsweise auf viele hundert Retard-Einzeldosen, z. B. in Form von sphäroiden Teilchen bzw. Pellets oder ausgerundeten Granulaten werden die Auswirkungen der unterschiedlichen Verweilzeiten und des unterschiedlichen pH-Wertes im Gastrointestinaltrakt auf die Blutspiegel nivelliert.

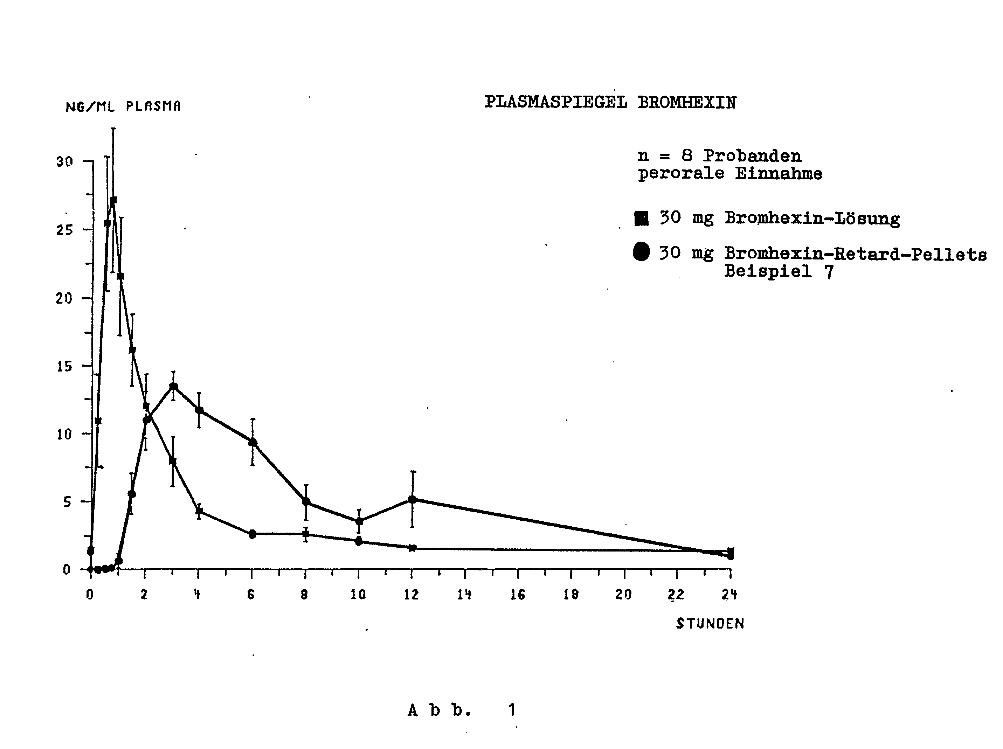

Aus der Abbildung 1 geht hervor, wie sich die Blutspiegel bei den erfindungsgemäß beschriebenen Retardformen von einer unverzögerten Form unterscheiden. Als unverzögerte Form wurde eine saure Lösung von Bromhexin gewählt, als Retardform beispielhaft Bromhexin-Pellets nach Beispiel 7.

Dargestellt sind die Mittelwertkurven von jeweils 8 Probanden. Die Blutplasmakonzentrationswerte von Bromhexin wurden nach entsprechender Aufarbeitung mittels Kapillar-Gaschromatographie bestimmt. Der Vergleich der Bromhexin-Plasmaspiegelverläufe nach peroraler Gabe von 30 mg Bromhexin als Lösung und der Pellet-Retardform zeigt, daß durch die Depotformulierung ein um ca. 1 Stunde verzögerter Beginn der Resorption und eine Verschiebung des Plasmaspiegelmaximums von ca. 0,75 Std. (Lösung) nach 3 Std. (Depot) erreicht wird. Die zwischen der 3. und 24. Stunde zum Teil deutlich höher liegenden Plasmaspiegel der Depotform beseisen, daß über viele Stunden eindeutig eine Resorption aus dem Darm stattfindet. Damit ist zum ersten Male der Beweis erbracht, daß trotz der praktischen Unlöslichkeit des Bromhexins im pH-Bereich des Intestinaltraktes durch geeignete Maßnahmen eine Resorption realisiert werden dann.

Für Retardformen außerordentlich ungewöhnlich ist der Vergleich der Flächen (AUC) unter den Plasmaspiegelkurven ($\bar{x} \pm S\,\bar{x}$):

|  | 0-12 Std. | 0-24 Std. |
|---|---|---|
| Lösung | 71,6 ± 24,4 | 88,1 ± 26,4 |
| Depot | 83,1 ± 19,9 | 119,4 ± 35,9 |

Bemerkenswerterweise ist somit die relative Bioverfügbarkeit der Depotform besser als die der normalerweise für eine Resorption als optimal geltende Wirkstofflösung!

Die Realisierung eines funktionierenden Bromhexin-Depots verlangt die Wahl der optimalen Retardform, der optimalen Säure, der optimalen Säuremenge, der optimalen Hüllenzusammensetzung und der optimalen Hüllendicke. Die besondere Schwierigkeit besteht darin, daß sich diese 5 Parameter nicht unabhängig voneinander variieren lassen, sondern sich gegenseitig beeinflussen.

Die nachfolgenden Beispiele sollen die Erfindung erläutern, ohne sie aber zu beschränken.

## Beispiel 1

10 kg ausgerundete Weinsäure-Starterkerne einer Teilchengröße zwischen 0,6 und 0,8 mm werden in einem rotierenden Kessel mit einer 10 %igen alkoholischen Polyvinylpyrrolidonlösung gleichmäßig befeuchtet, daraufhin wird solange ein fein pulverisiertes Gemisch aus

| | |
|---|---|
| Bromhexin | 8 Teile |
| Talcum | 2 Teile |

eingestreut, bis die Pellets wieder frei laufen. Nach einer kurzen Trockenphase sprüht man wieder Kleberlösung und trägt danach weiteres Pulver ein. Insgesamt werden auf diese Weise 3 kg der Pulbermischung eingetragen, wozu ca. 2,8 kg Kleberlösung notwendig sind. Die entsprechenden Wirkstoffpellets sind zwischen 0,7 und 0,9 mm groß und enthalten ca. 18 % Bromhexin und 75 % Weinsäure. Die Pellets werden nach der letzten Pulverauftragung gut getrocknet.

## Beispiel 2

30 kg nicht ausgerundete Zitronensäure-Starterkerne mit einer Teilchengröße von 0,5 bis 0,63 mm werden unter gleichen Bedingungen wie im Beispiel 1 angegeben mit 20 kg einer Pulvermischung aus 16 kg Bromhexinhydrochlorid und 4 kg Weinsäure auf eine Pelletgröße von 0,8 bis 1,12 mm gebracht. Als Starterkerne (jeweils 10 kg) wurden außerdem folgende Säuren verwendet : Ascorbinsäure, Fumarsäure, Äpfelsäure, Bernsteinsäure, Natrium- oder Kaliumhydrogensulfat, Monatrium- oder Monokaliumsalze der genannten mehrbasischen organischen Säuren.

Als Säurekomponente der aufzutragenden Mischung aus 8 Teilen Bromhexin bzw. Bromhexin-Salz und 2 Teilen Säure werden neben Weinsäure, Zitronensäure ebenfalls die oben genannten Säuren verwendet. Außerdem können auch Mischungen dieser Stoffe als saure Komponente Verwendung finden.

Das Verhältnis des auf die Kerne aufzutragenden Gemisches aus Bromhexin und sauer reagierender Komponente kann neben dem oben genannten von 8 : 2 auch folgende Werte aufweisen : 10 : 0, 9 : 1, 7 : 3, 6 : 4, 5 : 5, 4 : 6, 3 : 7, 2 : 8, 1 : 9.

## Beispiel 3

In einem Wirbelschichtgranuliergerät werden 10 kg Bromhexinpulver mit 22 kg Fumarsäurepulver gemischt. Mit Hilfe von 25 kg einer 5 %igen Hydroxypropylmethylcelluloselösung (Methylenchlorid/Isopropanol) wird bei langsamem Sprühen ein Aufbaugranulat hergestellt. 90 % des getrockneten, kugelartigen Granulates liegen zwischen 0,6 und 1,0 mm. Der Gehalt an Bromhexin beträgt ca. 30 %.

Anstelle von Fumarsäure finden auch Verwendung Zitronensäure, Ascorbinsäure, Weinsäure, Äpfelsäure, Bernsteinsäure, Mononatrium- und Monokaliumsalze der genannten mehrbasischen Säuren, Natrium- oder Kaliumhydrogensulfat.

Der Gehalt an Bromhexin kann durch Änderung der Zusammensetzung der Mischung auch die folgenden Werte aufweisen : 5 %, 10 %, 20 %, 40 %, 50 %.

## Beispiel 4

15 kg Zuckerkügelchen mit einem Durchmesser von 0,6-0,8 mm werden in einem rotierenden Kessel mit einer 5 %igen alkoholischen Hydroxypropylcelluloselösung gleichmäßig befeuchtet, daraufhin wird solange ein fein gepulvertes Gemisch aus 7 kg Bromhexincitrat, 2 kg Fumarsäure und 1 kg Talcum eingestreut, bis die Pellets wieder frei laufen. Nach Trocknen und Befeuchten trägt man wiederum Pulver ein. Zum Auftragen des gesamten Pulvers benötigt man ca. 12 kg Kleberlösung. Nach gründlichem Trocknen und Sieben werden die Bromhexin-Wirkstoffpellets mit einem 1 %igen Schutzfilm von Hydroxypropylmethylcellulose versehen.

## Beispiel 5

10 kg ausgerundete Weinsäure-Starterkerne einer Teilchengröße von 0,6-0,8 mm werden in einem rotierenden Kessel mit einer 12,5 %igen alkoholischen Lösung eines Methacrylsäure-Methacrylsäureester-Mischpolymerisats (Eudragit S[R]) besprüht. Insgesamt trägt man 1,5 % Lacktrockensubstanz auf. Die überzogenen Weinsäurepellets werden exakt nach Beispiel 1 mit Bromhexin und Talcum überzogen. Es wird soviel Pulver aufgetragen, daß der Wirkstoffgehalt 12 % beträgt.

## Beispiel 6

15 kg Bromhexinhydrochlorid, 55 kg Fumarsäure und 9 kg Polyvinylpyrrolidon werden 20 Minuten in einem Kubusmischer gemischt. Man fügt 0,4 kg Magnesiumstearat zu und gibt nach kurzen Mischen das Pulver über einen Walzenkompaktor, dem ein Trockengranuliergerät mit Siebeinrichtung nachgeschaltet ist. Verwendung findet die Fraktion 0,63 bis 1,0 mm. Feinere Anteile werden zurückgeführt und erneut verdichtet und gebrochen. Die abgesiebte Fraktion wird in einer Wirbelschichtapparatur durch Besprühen mit einer verdünnten Talcumpaste ausgerundet und anschließend mit einer 10 %igen Lacklösung, bestehend aus

| | |
|---|---|
| Methacrylsäure-Methacrylsäureester-Mischpolymerisat (Eudragit S[R]) | 8 Teile |
| Copolymerisat aus Acryl- und Methacrylsäureestern (Eudragit retard S[R]) | 2 Teile |

in Isopropanol/Aceton 7 : 3 aufgesprüht. Bezogen auf das Granulat wird 8 % Lack und 1 % Polyäthylenglykol 6 000 als Weichmacher aufgesprüht.

In-vitro-Wirkstofffreigabe Bromhexin :

Bedingungen : USP XX-paddle-Methode, 100 Umdrehungen/Minute, 37 °C.

1. Stunde   pH 1,2   USP-Magensaft
2-8. Stunde   pH 5,5   Phosphat-Puffer

Die Freigaben wurden stets unter diesen Bedingungen durchgeführt.

Ergebnis :

1. Stunde  25 % Bromhexin
2. Stunde  44 % Bromhexin
3. Stunde  59 % Bromhexin
4. Stunde  71 % Bromhexin
5. Stunde  80 % Bromhexin
6. Stunde  87 % Bromhexin
7. Stunde  93 % Bromhexin
8. Stunde  98 % Bromhexin

Beispiel 7

19 kg Bromhexin-Wirkstoffpellets nach Beispiel 1 werden in einem rasch umlaufenden Dragierkessel mit Schikanen mit einer Lösung von

| | |
|---|---|
| Methacrylsäure-Methacrylsäureester-Mischpolymerisat (Eudragit S$^R$) | 900 g |
| Hydroxypropylmethylcellulosephthalat (HP 55$^R$) | 900 g |

in 16,2 kg Isopropanol/Aceton 7 : 3 besprüht. Als Weichmacher wird 180 g Triacetin zugesetzt.
Nach dem Trocknen erhält man folgende Freigabewerte für Bromhexin :

1. Stunde 12,5 %
2. Stunde 36,2 %
3. Stunde 59,6 %
4. Stunde 69,5 %
5. Stunde 79,0 %
6. Stunde 88,1 %
7. Stunde 94,4 %

In der 2. und 3. Stunde ist eine deutliche Freigabebeschleunigung erkennbar.
In analoger Weise wurden auch Hüllen folgender Zusammensetzung auf die Pellets, die nach den Beispielen 1, 2 und 4 hergestellt wurden, aufgesprüht :

| | |
|---|---|
| A) Copolymerisat aus Acryl- und Methacrylsäureestern (Eudragit retard S$^R$) | 30 Teile |
| Methacrylsäure-Methacrylsäureester-Mischpolymerisat (Eudragit S$^R$) | 60 Teile |
| Polyäthylenglykol 6 000 | 10 Teile |
| | |
| B) Celluloseacetatphthalat | 30 Teile |
| Hydroxypropylmethylcellulosephthalat (HP 55$^R$) | 60 Teile |
| Triacetin | 10 Teile |
| | |
| C) Äthylcellulosephthalat | 70 Teile |
| Copolymerisat aus Acryl- und Methacrylsäureestern (Eudragit retard S$^R$) | 20 Teile |
| Triacetin | 10 Teile |
| | |
| D) Äthylcellulose | 10 Teile |
| Hydroxypropylmethylcellulosesuccinat | 80 Teile |
| Triacetin | 10 Teile |

Beispiel 8

12 kg Bromhexin-Wirkstoffpellets nach Beispiel 5 werden in einem rasch umlaufenden Drageekessel mit Schikanen mit einer Lösung von

| | |
|---|---|
| Methacrylsäure-Methacrylsäureester-Mischpolymerisat (Eudragit S$^R$) | 720 g |

in 6,48 kg Isopropanol besprüht.
Man erhält folgende Freigabewerte für Bromhexin :

| | |
|---|---|
| 1. Stunde | 3,2 % |
| 2. Stunde | 14,5 % |
| 3. Stunde | 35,1 % |
| 4. Stunde | 58,0 % |
| 5. Stunde | 74,8 % |

| | |
|---|---|
| 6. Stunde | 85,3 % |
| 7. Stunde | 94,1 % |
| 8. Stunde | 100,0 % |

Infolge der Isolierung des Weinsäure-Starterkernes tritt bei diesen Retardpellets erst ab der dritten Stunde eine Freigabebeschleunigung ein. Pellets dieser Art eignen sich in besonderem Maße zur Mischung mit unverzögerten Starterpellets.

Beispiel 9

Granulat nach Beispiel 3 wird nach Zumischung von 0,3 % Magnesiumstearat zu 6 mm bikonvexen, 100 mg schweren Tablettenkernen verpreßt.

5 kg dieser Kerne werden in einem Drageekessel mit einer 10 %igen Lösung (Aceton/Isopropanol) von

| | |
|---|---|
| Copolymerisat aus Acryl- und Methacrylsäure-estern (Eudragit retard S[R]) | 25 Teile |
| Hydroxypropylmethylcellulosephthalat (HP 55[R]) | 65 Teile |
| Triacetin | 10 Teile |

besprüht. Man trägt 6,5 % Lacktrockensubstanz auf die Kerne auf und erhält folgende Freigabewerte :

| | |
|---|---|
| 1. Stunde | 15,5 % |
| 2. Stunde | 35,3 % |
| 3. Stunde | 57,8 % |
| 4. Stunde | 70,0 % |
| 5. Stunde | 78,7 % |
| 6. Stunde | 85,3 % |
| 7. Stunde | 89,2 % |
| 8. Stunde | 93,4 % |

Obwohl diese Freigabewerte nahezu identisch sind mit den Werten der Pellets nach Beispiel 7 ergab die in-vivo-Prüfung dieser Retard-Tabletten stärker schwankende Bromhexin-Plasmaspiegel und eine im Vergleich zu den Pellets um ca. 25 % niedrigere relative Bioverfügbarkeit.

Beispiel 10

2 kg überzogene Bromhexin-Pellets nach Beispiel 6 werden mit 1,5 kg mikrokristalliner Cellulose, 0,4 kg Maisstärke und 0,1 kg Polyvinylpyrrolidon gemischt. Nach Zusatz von 20 g Magnesiumstearat wird noch für 5 Minuten nachgemischt. Aus der Mischung werden unter schwachem Preßdruck Tabletten gepreßt. Die Tabletten zerfallen in ca. 45 Sekunden ; die Bromhexin-Freigabe der einzelnen Retardpellets hat sich nur unwesentlich beschleunigt.

Beispiel 11

1,5 kg Bromhexinhydrochlorid, 1,0 kg Milchzucker, 0,5 kg Polyvinylpyrrolidon und 6,9 kg Polyacrylsäure (Handelsname Carbopol 940) werden mit Alkohol befeuchtet und durch ein Sieb, Maschenweite 1,5 mm, granuliert. Nach dem Trocknen mischt man 0,1 kg Magnesiumstearat zu und verpreßt das Granulat zu 9 mm-Matrix-Tabletten mit 200 mg Gewicht.

Man erhält folgende Freigabewerte :

| | | |
|---|---|---|
| 1. Stunde | 26,2 % | pH 1,2 |
| 2. Stunde | 29,7 % | pH 5,5 |
| 3. Stunde | 30,9 % | pH 5,5 |
| 4. Stunde | 32,1 % | pH 5,5 |
| 8. Stunde | 37,3 % | pH 5,5 |

**Ansprüche** (für die Vertragsstaaten : BE, CH, DE, FR, IT, LI, LU, NL, SE)

1. Neue Bromhexin-Retardformen, bestehend aus mit einem Überzug versehenen, sphäroiden Teilchen oder Tabletten, dadurch gekennzeichnet, daß sich die Retardformen aus Bromhexin und/oder dessen Salzen und aus Säuren, insbesondere organischen Genußsäuren im Verhältnis von mindestens 2 Mol Säure auf 1 Mol Bromhexin-Base oder mindestens 1 Mol Säure auf 1 Mol Bromhexin-Salz zusammensetzen, und einer, die sphäroiden Teilchen oder Tabletten umgebenden, aus 50-100 Gewichtsprozent säurenlöslichen, darmsaftlöslichen Lacken und aus 0-50 Gewichtsprozent eines magen- und darmsaftunlöslichen Copolymerisates aus Acryl- und Methacrylsäureestern bzw. 86-100 Gewichtsprozent säureunlöslichen, darmsaftlöslichen Lacken und 0-14 Gewichtsprozent Äthylcellulose aufgebauten Umhüllung besteht, wobei der Hüllenanteil 2 bis 30 Gewichtsprozent, vorzugsweise jedoch 3 bis 20 Gewichtsprozent, bezogen auf das Gewicht der sphäroiden Teilchen bzw. Tabletten beträgt.

9

2. Bromhexin-Retardformen nach Anspruch 1, dadurch gekennzeichnet, daß auf 1 Mol Bromhexin-Base bzw. Bromhexin-Salz bis zu 75 Mol Säure entfällt.

3. Bromhexin-Retardformen nach Anspruch 1 und 2 dadurch gekennzeichnet, daß die Gesamt-Wirkstoffdosis zwischen 5 und 100 mg Bromhexin bzw. Bromhexin-Salz liegt.

4. Bromhexin-Retardformen nach einem der vorhergehenden Ansprüche, dadurch gekennzeichnet, daß die sphäroiden Teilchen in Form ausgerundeter Granulate oder in Form von Pellets vorliegen und einen Durchmesser von 0,1 bis 3 mm aufweisen.

5. Bromhexin-Retardformen nach den Ansprüchen 1 bis 3, dadurch gekennzeichnet, daß die mit einem Diffusionslack überzogenen Tabletten einen Durchmesser von 4 bis 13 mm aufweisen.

6. Bromhexin-Retardformen nach einem der vorhergehenden Ansprüche, dadurch gekennzeichnet, daß die darmsaftlöslichen Lacke aus Methacrylsäure-Methacrylsäureester-Mischpolymerisate (Säurezahl 180 bis 200), Hydroxypropylmethylcellulosephthalat, Celluloseacetatphthalat, Äthylcellulosephthalat, Hydroxypropylmethylcellulosesuccinat, Celluloseacetatsuccinat, Hydroxypropylmethylcellulosehexahydrophthalat, Celluloseacetathexahydrophthalat, Hydroxypropylmethylcellulosetrimellitat, Methacrylsäure-Methacrylsäureester-Mischpolymerisat (Säurezahl 300 bis 330) für sich oder in Gemischen miteinander bestehen.

7. Bromhexin-Retardformen nach Anspruch 6, dadurch gekennzeichnet, daß der darmsaftlösliche Lack aus 50 Gewichtsprozent Methacrylsäure-Methacrylsäureester-Mischpolymerisat (Säurezahl 180 bis 200) und aus 50 Gewichtsprozent Hydroxypropylmethylcellulosphthalat besteht.

8. Bromhexin-Retardformen nach Anspruch 1 und 4, dadurch gekennzeichnet, daß die sphäroiden Teilchen einen aus Säure bestehenden zentralen Starterkern enthalten, welcher mit einer darmsaftlöslichen Lackkomponente versehen ist, auf den dann das Bromhexin bzw. Bromhexin-Salz aufgetragen sind.

9. Bromhexin-Retardformen nach Anspruch 1 bis 4 und 6 bis 8, dadurch gekennzeichnet, daß die sphäroiden Teilchen in Hartgelatine-Kapseln abgefüllt werden.

10. Bromhexin-Retardformen nach Anspruch 1 bis 4 und 6 bis 8, dadurch gekennzeichnet, daß die sphäroiden Teilchen, sofern sie einen Durchmesser von bis zu 1,5 mm aufweisen, mit üblichen Hilfsstoffen zu Tabletten verpreßt werden.

11. Bromhexin-Retardformen nach Anspruch 1 bis 4 und 6 bis 8, dadurch gekennzeichnet, daß für den Starterkern und das aufzutragende Gemisch auch verschiedene Säuren verwendet werden.

12. Verfahren zur Herstellung einer Bromhexin-Retardform gemäß den Ansprüchen 1 bis 11, dadurch gekennzeichnet, daß die sphäroiden Teilchen durch Auftragen von Bromhexin und/oder dessen Salzen mit Säure auf Starterkerne aus einer Säure oder sauren Substanz bzw. auf neutrale Starterkerne in Gegenwart eines Haftmittels gewonnen werden und hernach die die Umhüllung bildende Lacklösung aufgesprüht wird, bzw. Bromhexin und/oder dessen Salze mit sauren Substanzen granuliert, zu Tabletten verpreßt und die Tabletten mit einem Lack umhüllt werden, wobei in beiden Fällen der Lack die im Anspruch 1 angegebene Zusammensetzung aufweist.

**Ansprüche** (für den Vertragsstaat AT)

1. Verfahren zur Herstellung einer Bromhexin-Retardform, bestehend aus mit einem Überzug versehenen sphäroiden Teilchen oder Tabletten, dadurch gekennzeichnet, daß die sphäroiden Teilchen durch Auftragen von Bromhexin und/oder dessen Salzen mit Säure auf Starterkerne aus einer Säure oder sauren Substanz bzw. auf neutrale Starterkerne in Gegenwart eines Haftmittels gewonnen werden und hernach die die Umhüllung bildende Lacklösung aufgesprüht wird, bzw. Bromhexin und/oder dessen Salze mit sauren Substanzen granuliert, zu Tabletten verpreßt und die Tabletten mit einem Lack umhüllt werden, wobei in beiden Fällen der Lack aus 50-100 Gewichtsprozent säureunlöslichen, darmsaftlöslichen Lacken und aus 0-50 Gewichtsprozent eines magen- und darmsaftunlöslichen Copolymerisates aus Acryl- und Methacrylsäureestern bzw. 86-100 Gewichtsprozent säureunlöslichen, darmsaftlöslichen Lacken und 0-14 Gewichtsprozent Äthylcellulose besteht und der Hüllenanteil 2 bis 30 Gewichtsprozent, vorzugsweise jedoch 3 bis 20 Gewichtsprozent, bezogen auf das Gewicht der sphäroiden Teilchen bzw. Tabletten beträgt.

2. Verfahren zur Herstellung einer Bromhexin-Retardform nach Anspruch 1, dadurch gekennzeichnet, daß auf 1 Mol Bromhexin-Base bzw. Bromhexin-Salz bis zu 75 Mol Säure verwendet wird.

3. Verfahren zur Herstellung einer Bromhexin-Retardform nach Anspruch 1 und 2, dadurch gekennzeichnet, daß die verwendete Gesamt-Wirkstoffdosis zwischen 5 und 100 mg Bromhexin bzw. Bromhexin-Salz liegt.

4. Verfahren zur Herstellung einer Bromhexin-Retardform nach einem der Ansprüche 1 bis 3, dadurch gekennzeichnet, daß die sphäroiden Teilchen in Form ausgerundeter Granulate oder in Form vor Pellets verwendet werden und einen Durchmesser von 0,1 bis 3 mm aufweisen.

5. Verfahren zur Herstellung einer Bromhexin-Retardform nach einem der Ansprüche 1 bis 4, dadurch gekennzeichnet, daß die darmsaftlöslichen Lacke aus Methacrylsäure-Methacrylsäureester-Mischpolymerisate (Säurezahl 180 bis 200), Hydroxypropylmethylcellulosephthalat, Celluloseacetatphthalat, Äthylcellulosephthalat, Hydroxypropylmethylcellulosesuccinat, Celluloseacetatsuccinat, Hydroxypropylmethylcellulosehexahydrophthalat, Celluloseacetathexahydrophthalat, Hydroxypropylmethylcellulosetrimellitat, Methacrylsäure-Methacrylsäureester-Mischpolymerisat (Säurezahl 300 bis 330) für sich oder in Gemischen miteinander bestehen.

6. Verfahren zur Herstellung einer Bromhexin-Retardform nach Anspruch 5, dadurch gekennzeichnet, daß der darmsaftlösliche Lack aus 50 Gewichtsprozent Methacrylsäure-Methacrylsäureester-Mischpolymerisat (Säurezahl 180 bis 200) und aus 50 Gewichtsprozent Hydroxypropylmethylcellulosephthalat besteht.

7. Verfahren zur Herstellung einer Bromhexin-Retardform nach Anspruch 1 und 4, dadurch gekennzeichnet, daß die sphäroiden Teilchen einen aus Säure bestehenden zentralen Starterkern enthalten, welcher mit einer darmsaftlöslichen Lackkomponente versehen ist, auf den dann das Bromhexin bzw. Bromhexin-Salz aufgetragen sind.

8. Verfahren zur Herstellung einer Bromhexin-Retardform nach Anspruch 1 bis 7, dadurch gekennzeichnet, daß die sphäroiden Teilchen in Hartgelatine-Kapseln abgefüllt werden.

9. Verfahren zur Herstellung einer Bromhexin-Retardform nach Anspruch 1 bis 7, dadurch gekennzeichnet, daß die sphäroiden Teilchen, sofern sie einen Durchmesser von bis zu 1,5 mm aufweisen, mit üblichen Hilfsstoffen zu Tabletten verpreßt werden.

10. Verfahren zur Herstellung einer Bromhexin-Retardform nach Anspruch 1 bis 7, dadurch gekennzeichnet, daß für den Starterkern und das aufzutragende Gemisch auch verschiedene Säuren verwendet werden.

**Claims** (for the Contracting States : BE, CH, DE, FR, IT, LI, LU, NL, SE)

1. New delayed-release forms of bromhexine, consisting of spheroid particles or tablets provided with a coating, characterised in that the delayed-release forms are made up of bromhexine and/or the salts thereof and acids, particularly edible organic acids, in a ratio of at least 2 moles of acid per mole of bromhexine base or at least 1 mole of acid to 1 mole of bromhexine salt, and a coating surrounding the spheroid articles or tablets and made up to 50 to 100 % by weight of lacquers which are insoluble in acid and soluble in intestinal juice and 0 to 50 % by weight of a copolymer of acrylic and methacrylic acid esters insoluble in gastric and intestinal juices, or 86 to 100 % by weight of lacquers insoluble in acid and soluble in intestinal juices and 0 to 14 % by weight of ethyl cellulose, whilst the coating constitutes from 2 to 30 % by weight, but preferably from 3 to 20 % by weight, relative to the weight of the spheroid particles or tablets.

2. Delayed-release bromhexine forms as claimed in claim 1, characterised in that there are up to 75 moles of acid per 1 mole of bromhexine base or bromhexine salt.

3. Delayed-release bromhexine forms as claimed in claims 1 and 2, characterised in that the total dosage of active substance is between 5 and 100 mg of bromhexine or bromhexine salt.

4. Delayed-release bromhexine forms as claimed in one of the preceding claims, characterised in that the spheroid particles take the form of rounded granules or pellets and have a diameter of from 0.1 to 3 mm.

5. Delayed-release bromhexine forms as claimed in claims 1 to 3, characterised in that the tablets coated with a diffusion lacquer have a diameter of from 4 to 13 mm.

6. Delayed-release bromhexine forms as claimed in one of the preceding claims, characterised in that the lacquers soluble in intestinal juices consist of methacrylic acid/methacrylic acid ester copolymers (acid number 180-200), hydroxypropylmethylcellulose phthalate, cellulose acetate phthalate, ethylcellulose phthalate, hydroxypropylmethylcellulose succinate, cellulose acetate succinate, hydroxypropylmethylcellulose hexahydrophthalate, cellulose acetate hexahydrophthalate, hydroxypropylmethylcellulose trimellitate, methacrylic acid/methacrylic acid ester copolymer (acid number 300-330), either as such or in admixture with one another.

7. Delayed-release bromhexine forms as claimed in claim 6, characterised in that the lacquer soluble in the intestinal juices consists of 50 % by weight of methacrylic acid/methacrylic acid ester copolymer (acid number 180-200) and 50 % by weight of hydroxypropylmethyl cellulose phthalate.

8. Delayed-release bromhexine forms as claimed in claims 1 and 4, characterised in that the spheroid particles contain a central starter core consisting of acid, which is provided with a lacquer component soluble in intestinal juices, to which the bromhexine or bromhexine salt is then applied.

9. Delayed-release bromhexine forms as claimed in claims 1 to 4 and 6 to 8, characterised in that the spheroid particles are packed into hard gelatine capsules.

10. Delayed-release bromhexine forms as claimed in claims 1 to 4 and 6 to 8, characterised in that the spheroid particles which have a diameter of up to 1.5 mm are compressed with conventional excipients to form tablets.

11. Delayed-release bromhexine forms as claimed in claims 1 to 4 and 6 to 8, characterised in that different acids are also used for the starter core and for the mixture which is to be applied.

12. Process for the preparation of a delayed-release bromhexine form as claimed in claims 1 to 11, characterised in that the spheroid particles are obtained by applying bromhexine and/or a salt thereof with an acid to starter cores consisting of an acid or acidic substance or to neutral starter cores, in the presence of an adhesive, and then the lacquer solution which forms the coating is sprayed on, or bromhexine and/or a salt thereof is granulated with acidic substances, compressed to form tablets and the tablets are coated with a lacquer, whilst in both cases the lacquer has the composition recited in claim 1.

**Claims** (for the Contracting State AT)

1. Process for the preparation of a delayed-release bromhexine form, consisting of spheroid particles or tablets provided with a coating, characterised in that the spheroid particles are prepared by applying bromhexine and/or a salt thereof with an acid to starter cores consisting of an acid or acidic substance or to neutral starter cores in the presence of an adhesive, and then the lacquer solution which forms the coating is sprayed on, or bromhexine and/or a salt thereof is granulated with acidic substances, compressed to form tablets and the tablets are coated with a lacquer, whilst in both cases the lacquer consists of 50 to 100 % by weight of lacquers which are insoluble in acid and soluble in intestinal juices and of 0-50 % by weight of a copolymer of acrylic and methacrylic acid esters insoluble in gastric and intestinal juices, or 86-100 % by weight of lacquers insoluble in acid and soluble in intestinal juices and 0-14 % by weight of ethyl cellulose and the coating constitutes from 2 to 30 % by weight, but preferably 3 to 20 % by weight, relative to the weight of the spheroid particles or tablets.

2. Process for the preparation of a delayed-release bromhexine forms as claimed in claim 1, characterised in that there are used up to 75 moles of acid per 1 mole of bromhexine base or bromhexine salt.

3. Process for the preparation of delayed-release bromhexine forms as claimed in claims 1 and 2, characterised in that the total dosage of active substance is between 5 and 100 mg of bromhexine or bromhexine salt.

4. Process for the preparation of delayed-release bromhexine forms as claimed in one of claims 1 to 3, characterised in that the spheroid particles are used in the form of rounded granules or pellets and have a diameter of from 0.1 to 3 mm.

5. Process for the preparation of delayed-release bromhexine forms as claimed in one of claims 1 to 4, characterised in that the lacquers soluble in intestinal juices consist of methacrylic acid/methacrylic acid ester copolymers (acid number 180-200), hydroxypropylmethylcellulose phthalate, cellulose acetate phthalate, ethylcellulose phthalate, hydroxypropylmethylcellulose succinate, cellulose acetate succinate, hydroxypropylmethylcellulose hexahydrophthalate, cellulose acetate hexahydrophthalate, hydroxypropylmethylcellulose trimellitate, methacrylic acid/methacrylic acid ester copolymer (acid number 300-330), either as such or in admixture with one another.

6. Process for the preparation of delayed-release bromhexine forms as claimed in claim 5, characterised in that the lacquer soluble in the intestinal juices consists of 50 % by weight of methacrylic acid/methacrylic acid ester copolymer (acid number 180-200) and 50 % by weight of hydroxypropylmethyl cellulose phthalate.

7. Process for the preparation of delayed-release bromhexine forms as claimed in claims 1 and 4, characterised in that the spheroid particles contain a central starter core consisting of acid, which is provided with a lacquer component soluble in intestinal juices, to which the bromhexine or bromhexine salt is then applied.

8. Process for the preparation of delayed-release bromhexine forms as claimed in claims 1 to 7, characterised in that the spheroid particles are packed into hard gelatine capsules.

9. Process for the preparation of delayed-release bromhexine forms as claimed in claims 1 to 7, characterised in that the spheroid particles which have a diameter of up to 1.5 mm are compressed with conventional excipients to form tablets.

10. Process for the preparation of delayed-release bromhexine forms as claimed in claims 1 to 7, characterised in that different acids are also used for the starter core and for the mixture which is to be applied.

**Revendications** (pour les Etats contractants : BE, CH, DE, FR, IT, LI, LU, NL, SE)

1. Nouvelles formes retard de bromhexine constituées de particules sphéroïdes ou de comprimés munis d'un revêtement, caractérisées en ce que les formes retard se composent de bromhexine et/ou de ses sels et d'acides, en particulier d'acides alimentaires organiques, dans la proportion d'au moins 2 moles d'acide pour 1 mole de bromhexine-base ou d'au moins 1 mole d'acide pour 1 mole de bromhexine-sel et d'un enrobage entourant les particules sphéroïdes ou les comprimés, formé de 50-100 % en poids de vernis solubles dans le suc intestinal, insolubles dans les acides et de 0-50 % en poids d'un copolymérisat insoluble dans le suc gastrique et le suc intestinal, d'esters d'acide acrylique et d'acide méthacrylique ou respectivement de 86-100 % en poids de vernis solubles dans le suc intestinal, insolubles dans les acides et de 0-14 % en poids d'éthylcellulose, la proportion de l'enveloppe représentant 2 à 30 % en poids, de préférence cependant 3 à 20 % en poids par rapport au poids des particules sphéroïdes ou respectivement des comprimés.

2. Formes retard de bromhexine selon la revendication 1, caractérisées en ce qu'à 1 mole de bromhexine-base ou respectivement de bromhexine-sel correspondent jusqu'à 75 moles d'acide.

3. Formes retard de bromhexine selon la revendication 1 et 2, caractérisées en ce que la dose totale de substance active est comprise entre 5 et 100 mg de bromhexine ou respectivement de bromhexine-sel.

4. Formes retard de bromhexine selon l'une des revendications précédentes, caractérisées en ce que les particules sphéroïdes se présentent sous forme de granulés arrondis ou sous forme de boulettes et présentent un diamètre de 0,1 à 3 mm.

5. Formes retard de bromhexine selon les revendications 1 à 3, caractérisées en ce que les comprimés revêtus d'un vernis de diffusion présentent un diamètre de 4 à 13 mm.

6. Formes retard de bromhexine selon l'une des revendications précédentes, caractérisées en ce que les vernis solubles dans le suc intestinal sont constitués de copolymérisats d'acide méthacrylique-ester d'acide méthacrylique (indice d'acide 180 à 200), de phtalate d'hydroxypropylméthylcellulose, d'acéto-phtalate de cellulose, de phtalate d'éthylcellulose, de succinate d'hydroxypropylméthylcellulose d'acéto-succinate de cellulose, d'hexahydrophtalate d'hydroxypropylméthylcellulose, d'acétohexahydrophtalate de cellulose, de trimellitate d'hydroxypropylméthylcellulose de copolymérisat d'acide méthacrylique-ester d'acide méthacrylique (indice d'acide 300 à 330) en soi ou en mélanges les uns avec les autres.

7. Formes retard de bromhexine selon la revendication 6, caractérisées en ce que le vernis soluble dans le suc intestinal est constitué de 50 % en poids de copolymérisat d'acide méthacrylique-ester d'acide méthacrylique (indice d'acide 180 à 200) et de 50 % en poids de phtalate d'hydroxypropylméthylcellulose.

8. Formes retard de bromhexine selon les revendications 1 et 4, caractérisées en ce que les particules sphéroïdes contiennent un noyau de départ central constitué d'acide, lequel est muni d'un composant de vernis soluble dans le suc intestinal, sur lequel sont ensuite appliquées la bromhexine ou respectivement la bromhexine-sel.

9. Formes retard de bromhexine selon la revendication 1 à 4 et 6 à 8, caractérisées en ce que les particules sphéroïdes sont introduites dans des capsules en gélatine dure.

10. Formes retard de bromhexine selon la revendication 1 à 4 et 6 à 8, caractérisées en ce que les particules sphéroïdes, dans la mesure où elles présentent un diamètre allant jusqu'à 1,5 mm, sont pressées en comprimés avec des adjuvants usuels.

11. Formes retard de bromhexine selon la revendication 1 à 4 et 6 à 8, caractérisées en ce que pour le noyau de départ et le mélange à appliquer, on utilise également des acides différents.

12. Procédé pour la préparation d'une forme retard de bromhexine selon les revendications 1 à 11, caractérisé en ce que les particules sphéroïdes sont obtenues par application de bromhexine et/ou de ses sels avec des acides sur des noyaux de départ constitués d'un acide ou d'une substance acide ou respectivement sur des noyaux de départ neutres en présence d'un agent adhésif et qu'ensuite la solution de vernis formant l'enrobage est pulvérisée, ou respectivement la bromhexine et/ou ses sels sont granulés avec des substances acides, pressés en comprimés et les comprimés sont enrobés avec un vernis, dans les deux cas le vernis présentant la composition indiquée dans la revendication 1.

**Revendications** (pour l'Etat contractant AT)

1. Procédé pour la préparation d'une forme retard de bromhexine constituée de particules sphéroïdes ou de comprimés munis d'un revêtement, caractérisé en ce que les particules sphéroïdes sont obtenues par application de bromhexine et/ou de ses sels avec des acides sur des noyaux de départ constitués d'un acide ou d'une substance acide ou respectivement sur des noyaux de départ neutres en présence d'un agent adhésif et ensuite, la solution de vernis formant l'enrobage est pulvérisée, ou respectivement la bromhexine et/ou ses sels sont granulés avec des substances acides, pressés en comprimés et les comprimés sont enrobés d'un vernis, dans les deux cas le vernis étant constitué de 50-100 % en poids de vernis solubles dans le suc intestinal, insolubles dans les acides et de 0-50 % en poids d'un copolymérisat d'esters d'acide acrylique et d'acide méthacrylique, insoluble dans le suc gastrique et dans le suc intestinal ou respectivement de 86-100 % en poids de vernis solubles dans le suc intestinal, insolubles dans les acides et de 0-14 % en poids d'éthylcellulose et la proportion d'enveloppe étant de 2 à 30 % en poids, de préférence cependant de 3 à 20 % en poids par rapport au poids des particules sphéroïdes ou respectivement des comprimés.

2. Procédé pour la préparation d'une forme retard de bromhexine selon la revendication 1, caractérisé en ce que pour 1 mole de bromhexine-base ou respectivement de bromhexine-sel, on utilise jusqu'à 75 moles d'acide.

3. Procédé pour la préparation d'une forme retard de bromhexine selon les revendications 1 et 2, caractérisé en ce que la dose totale de substance active utilisée est comprise entre 5 et 100 mg de bromhexine ou respectivement de bromhexine-sel.

4. Procédé pour la préparation d'une forme retard de bromhexine selon l'une des revendications 1 à 3, caractérisé en ce que les particules sphéroïdes sont utilisées sous forme de granulés arrondis ou sous forme de boulettes et présentent un diamètre de 0,1 à 3 mm.

5. Procédé pour la préparation d'une forme retard de bromhexine selon l'une des revendications 1 à 4, caractérisé en ce que les vernis solubles dans le suc intestinal sont constitués de copolymérisats d'acide méthacrylique-ester, d'acide méthacrylique (indice d'acide 180 à 200), de phtalate d'hydroxypropylméthylcellulose, d'acétophtalate de cellulose, de phtalate d'éthylcellulose, de succinate d'hydroxypropylméthylcellulose, d'acétosuccinate de cellulose, d'hexahydrophtalade d'hydroxypropylméthylcellulose, d'acétohexahydrophtalate de cellulose, de trimellitate d'hydroxypropylméthylcellulose, de copolymérisat d'acide méthacrylique-ester d'acide méthacrylique (indice d'acide 300 à 330) en soi ou en mélanges les uns avec les autres.

6. Procédé pour la préparation d'une forme retard de bromhexine selon la revendication 5, caractérisé en ce que le vernis soluble dans le suc intestinal est constitué de 50 % en poids de copolymérisat d'acide méthacrylique-ester d'acide méthacrylique (indice d'acide 180 à 200) et de 50 % en poids de phtalate d'hydroxypropylméthylcellulose.

7. Procédé pour la préparation d'une forme retard de bromhexine selon les revendications 1 et 4, caractérisé en ce que les particules sphéroïdes contiennent un noyau de départ central constitué d'acide, lequel est muni d'un composant de vernis soluble dans le suc intestinal, sur lequel sont ensuite appliquées la bromhexine ou respectivement la bromhexine-sel.

8. Procédé pour la préparation d'une forme retard de bromhexine selon la revendication 1 à 7, caractérisé en ce que les particules sphéroïdes sont introduites dans des capsules en gélatine dure.

9. Procédé pour la préparation d'une forme retard de bromhexine selon la revendication 1 à 7, caractérisé en ce que les particules sphéroïdes, dans la mesure où elles présentent un diamètre allant jusqu'à 1,5 mm, sont pressées en comprimés avec des adjuvants usuels.

10. Procédé pour la préparation d'une forme retard de bromhexine selon la revendication 1 à 7, caractérisé en ce que pour le noyau de départ et le mélange à appliquer on utilise également des acides différents.

NG/ML PLASMA

PLASMASPIEGEL BROMHEXIN

n = 8 Probanden
perorale Einnahme

■ 30 mg Bromhexin-Lösung

● 30 mg Bromhexin-Retard-Pellets
Beispiel 7

STUNDEN

0 069 259

A b b.    1